# EUROPÄISCHE PATENTSCHRIFT

(11) **EP 1 183 043 B1**
(45) Veröffentlichungstag und Bekanntmachung des Hinweises auf die Patenterteilung: **07.04.2004**
(21) Anmeldenummer: 00936524.8
(22) Anmeldetag: 19.05.2000
(51) Int. Cl.: A61K 38/57, A61K 38/48, A61K 38/45, A61K 38/36

(54) **ARZNEIMITTEL ZUR LOKALEN ANWENDUNG, DAS FIBRINOGEN, THROMBIN, TRANSGLUTAMINASEN UND PROTEINASEINHIBITOREN ENTHÄLT**
MEDICAMENT FOR LOCALISED APPLICATION, CONTAINING FIBRINOGEN, THROMBIN, TRANSGLUTAMINASES AND PROTEINASE INHIBITORS
MEDICAMENT POUR APPLICATION LOCALE, CONTENANT DU FIBRINOGENE, DE LA THROMBINE, DES TRANSGLUTAMINASES ET DES INHIBITEURS DE PROTEASE

(30) Priorität: 19.05.1999 AT 89599
(43) Veröffentlichungstag der Anmeldung: 06.03.2002
(60) Teilanmeldung: 02015881.2
(73) Patentinhaber: Bio & Bio Licensing SA, 1425 Luxembourg (LU)
(72) Erfinder: EIBL, Johann, A-1180 Wien (AT)
(74) Vertreter: Schwarz, Albin, Dr.
(86) Internationale Anmeldenummer: PCT/AT2000/000141
(87) Internationale Veröffentlichungsnummer: WO 2000/071153

(56) Entgegenhaltungen:
- EP-A- 0 534 178
- WO-A-99/11301
- US-A- 4 427 650
- US-A- 4 442 655
- US-A- 5 514 579

## Beschreibung

Der Wundverschluß und die damit im Zusammenhang stehende Blutstillung erfolgt physiologisch durch Gerinnung des austretenden Blutes im Wundbett, wodurch der Verschluß von kleinen Blutgefäßen und Kapillaren zustandekommt. Die danach einsetzende Wundheilung erfolgt mit Hilfe der durch das geronnene Blut gebildeten, provisorischen extrazellulären Matrix (ECM) (Clark, R.A.F. et al., 1982, J. Invest. Dermatol. 70:264-269; Clark, RA.F.(ed.), 1996, The Molecular and Cellular Biology of Wound Repair, Plenum Press, New York). Diese Matrix besteht, abgesehen von Blutzellen, im wesentlichen aus Fibrin als Gerüstsubstanz, die als Reservoir für eine Reihe von Plasmaproteinen dient, die für die einsetzende Wundheilung von Bedeutung sind, wie Fibronektin (Mosesson, M.W. und Umfleet, R., 1970, J. Biol. Chem. 245:5726-5736; Clark, R.A.F. et al., 1982, J. Invest. Dermatol. 70:264-269), Vitronektin (Preissner, K.T. und Jenne, D., 1991, Thromb. Haemost. 66:189-194), Plasminogen (Castellino,F.J. et al., 1983, Ann. NY Acad. Sci. 408:595-601), Plasminogenaktivator (Thorsen, S. et al., 1972, Thromb. Pathol. Haemost. 28:65-74), Plasminogenaktivator-Inhibitor (Wagner, O.F. et al., 1989, Blood 70:1645-1653), und alpha₂-Plasmininhibitor (Sakata, Y. und Aoki, N., 1980, J. Clin. Invest. 65:290-297).

Die Menge von Plasminogenaktivator, Plasminogenaktivator-Inhibitor und alpha₂-Plasmininhibitor und das Verhältnis zueinander üben eine wesentliche Kontrolle auf den nachfolgenden Abbauprozeß des Fibrins aus. In diesem Fibringerüst sind aber noch andere Substanzen, wie z.B. Thrombin, TGF-beta und PDGF enthalten, die für die Einwanderung von Zellen und die mehrfache Remodellierung (Umbau) der provisorischen ECM zu der endgültigen ECM mit den entsprechenden Zellpopulationen erforderlich sind.

Als erstes wandern in größerer Menge Granulozyten in das Wundgebiet und in den Wundverschluß ein. Sie setzen verschiedene, für den einsetzenden Wundheilungsvorgang wichtige Substanzen, insbesondere Kollagenasen und Elastasen, frei und zerstören extrazellulär und intrazellulär Mikroorganismen, die ins Wundgebiet gelangt sind und die sich dort vermehren können. Während die Einwanderung von Granulozyten zu Ende geht, treten im Wundgebiet vermehrt Monozyten inklusive Makrophagen auf. Am dritten Tag kommt es zum Aussprossen von Fibroblasten im Wundgebiet, die über Fibronektinstränge bis an die Oberfläche des Wundverschlusses gelangen. Gefolgt von der Einsprossung von Blutkapillaren kommt es zu einer Reihe von Zelltransformationen und Remodellierungsprozessen, die in den meisten Fällen wieder zur vollständigen Integrität des verletzten Gewebes führen (Clark, R.A.F.(ed.), 1996, The Molecular and Cellular Biology of Wound Repair, Plenum Press, New York).

Den ersten Versuchen, die bereits 80 bis 90 Jahre zurückliegen, mit Hilfe von Blutplasma Wunden zu verschließen, waren wegen der relativ niedrigen Viskosität im Vergleich zu Blut, der geringen Adhärenz im Wundbett und wegen der hohen Fragilität eines sich allenfalls aus Plasma ausbildenden Clots von geronnenem Plasma kein Erfolg beschieden.

Die Verwendung von angereicherten Fibrinogenlösungen anstelle von Plasma zur Blutstillung und zum Wundverschluß war anfangs ebenfalls ohne Erfolg, doch schließlich konnte durch Anhebung der Fibrinogenkonzentration solcher fibrinogenhältiger Lösungen um mehr als das 10-fache des Fibrinogenspiegels im Plasma ein wesentlicher Erfolg erzielt werden (Löblich, 1975, unpublizierte Mitteilung).

Bei der Umwandlung von Fibrinogen in Fibrin besteht die Möglichkeit, daß der hämostatisch wirksame Fibrinwundverschluß sich nach einigen Stunden durch Wundbettenzyme ablöst und es dadurch zur Nachblutung kommt. Die Ablösung des Fibrinwundverschlusses vom Wundbett ist der wesentlich häufigere und daher gefährlichere Vorgang als die Fibrinolyse des gesamten Fibrinwundverschlusses.

Schon bei den ersten erfolgreichen Anwendungen von hochkonzentrierten Fibrinogenlösungen (Matras, H. et al., 1972, Wr. Med. Wschr. 122:517-523) und der Umwandlung von Fibrinogen in Fibrin durch Thrombin im Wundgebiet zeigte sich, daß die Abhebung des Fibrinwundverschlusses und die damit meist eintretenden Nachblutungen durch Fibrinolyseinhibitoren vermieden werden kann. Von den versuchten niedermolekularen Inhibitoren konnten Epsilonaminokapron-Säure und Derivate als wirksam befunden werden, allerdings wiesen sie den Nachteil auf, schnell aus dem geronnenen Fibrin und aus dem Wundgebiet zu diffundieren und somit ihre lokale Wirksamkeit zu verlieren.

Die Zumischung von hochmolekularen Inhibitoren, wie z.B. Aprotinin (Trasylol), konnte erfolgreich durchgeführt werden, indem es nur zu einer langsamen Diffusion des Inhibitors aus dem Wundgebiet kommt, allerdings mit dem Nachteil, daß es sich hierbei um einen bovinen und somit xenogenen Proteaseinhibitor handelt, der potentiell Allergien und Anaphylaxien hervorrufen kann. Neuerdings sind auch wegen der potentiellen Übertragungsfähigkeit von Zoonosen Einwände gegen die Verwendung tierischen Materials für die parenterale Anwendung beim Menschen gemacht worden.

In der WO-A - 99/11301 wird der Vorschlag gemacht, anstelle von Aprotinin Elastaseinhibitoren oder andere gegen Leukozytenproteasen wirksame Inhibitoren zu verwenden. Dieser Vorschlag bewirkt jedoch nach Erkenntnis des Erfinders der gegenständlichen Erfindung verschiedene Nachteile. Zwar sind diese Inhibitoren direkt oder indirekt durch Inhibition von Enzymen, die das fibrinolytische System aktivieren können, wirksam, sie können jedoch durch starke Hemmung der von den Granulozyten freigesetzten Proteasen, wie Kollagenasen und Elastasen, die Einleitung der Wundheilung nach Einwanderung der Granulozyten in das Wundgebiet stören.

Eine weitere Schwierigkeit bei der Herstellung von fibrinogen- und thrombinhältigen pharmazeutischen Arzneimitteln, die einen allogenen Proteaseinhibitor und ein Transglutaminasezymogen enthalten, ist durch die seit längerer Zeit erforderliche Virusinaktivierung solcher Zubereitungen gegeben. Bei der Virusinaktivierung geht meist ein großer Teil der Aktivität des in der Zubereitung enthaltenen Proteaseinhibitors bzw. Transglutaminasezymogens verloren, so daß die nach Durchführung des Virusinaktivierungsverfahrens erhaltenen Zubereitungen oft nur mehr eine geringe Aktivität des Proteaseinhibitors bzw. Transglutaminasezymogens aufweisen. Dadurch kann es zur unzureichenden Hemmung von im Wundbett vorhandenen fibrinolytischen Enzymen und in der Folge zur Ablösung des Fibrinwundverschlusses vom Wundbett kommen.

Die vorliegende Erfindung stellt sich daher die Aufgabe, ein Arzneimittel zur Verfügung zu stellen, das lokal zum Zwecke der Blutstillung und/oder des Wundverschlusses und/oder der Wundheilungsförderung angewendet werden kann, wobei die Verwendung eines xenogenen Proteaseinhibitors vermieden werden und dennoch eine ausreichende Hemmung fibrinolytischer Enzyme im Wundbett nach Aufbringen des Arzneimittels gegeben sein soll, so daß es zu keiner Ablösung des Fibrinwundverschlusses vom Wundbett kommt. Weiters soll die Störung der Einleitung der Wundheilung infolge einer Hemmung der von den ins Wundgebiet eingewanderten Granulozyten freigesetzten Proteasen, wie Kollagenasen und Elastasen, durch das Arzneimittel möglichst vermieden werden.

Diese Aufgabe wird für ein Arzneimittel, das als Wirkstoffe - konventionell aus allogenem Plasma oder Gewebe oder gentechnologisch hergestellt - Fibrinogen bzw. Fibrin, Thrombin und eine oder mehrere Transglutaminasen aufweist, dadurch gelöst, daß das Arzneimittel als weiteren Wirkstoff einen oder mehrerere Proteaseinhibitoren enthält, die aus der Gruppe der Serpine, die keine inhibierende Wirkung auf Kollagenasen und Elastasen besitzen, ausgewählt sind, wobei alle Wirkstoffe allogenen Ursprungs und einem Verfahren zur Virusabreicherung und/oder Virusinaktivierung unterzogen sind, mit der Maßgabe, daß die Virusinaktivierung des einen oder der mehreren Proteaseinhibitoren nicht in Gegenwart der anderen Wirkstoffe durchgeführt worden ist.

Durch die Verwendung von Serpinen, die keine inhibierende Wirkung auf Kollagenasen und Elastasen besitzen, als Proteaseinhibitoren wird die Hemmung der von den ins Wundgebiet eingewanderten Granulozyten freigesetzten Proteasen weitgehend vermieden, so daß die Einleitung der Wundheilung durch das erfindungsgemäße Arzneimittel nicht beeinträchtigt wird.

Die vorliegende Erfindung beruht auch auf der Erkenntnis, daß die inhibitorische Aktivität allogener Proteaseinhibitoren wesentlich besser erhalten bleibt, wenn diese nicht in einer Zubereitung, welche einen oder mehrere der anderen Wirkstoffe des Arzneimittels enthält, der Virusinaktivierung unterworfen werden, sondern getrennt von den anderen Wirkstoffen virusinaktiviert werden. Auf diese Weise ist es möglich, erfindungsgemäße Arzneimittel herzustellen, die virusinaktivierte allogene Proteaseinhibitoren mit ausreichender Aktivität enthalten, so daß fibrinolytische Enzyme im Wundbett nach Aufbringen des Arzneimittels gehemmt werden und eine Ablösung des Fibrinwundverschlusses vom Wundbett verhindert werden kann.

In diesem Zusammenhang wird darauf hingewiesen, daß der Begriff "Virusinaktivierung", wie er für die Zwecke der vorliegenden Erfindung gebraucht ist, keine Verfahren umfaßt, die lediglich eine Virusabreicherung bewirken.

Das erfindungsgemäße Arzneimittel enthält als Wirkstoff unter anderem Fibrinogen bzw. Fibrin. Darunter ist zu verstehen, daß im Arzneimittel je nach Anwendungsform entweder Fibrinogen als solches oder aus Fibrinogen durch Einwirkung von Thrombin entstandenes Fibrin vorliegt.

Gegenstand der Erfindung ist somit ein zusammengesetztes Arzneimittel, das eine sterile, virussichere, langsam resorbierbare und remodellierbare allogene, provisorische extrazelluläre Matrix darstellt bzw. eine solche ausbildet, die lokal angewendet werden kann oder sich erst lokal ausbildet. Da die Wirkstoffe dieser Arzneimittel hochmolekularen Charakter besitzen, und damit potentielle Antigene sind, werden nur allogene Wirkstoffe - bezogen auf die Spezies, bei der das Arzneimittel angewendet werden soll - bei der Herstellung dieser Arzneimittel verwendet.

Die allogene, provisorische extrazelluläre Matrix ermöglicht bei ihrer Herstellung bzw. Aufbringung durch Zusätze von weiteren allogenen, virussicheren Wirkstoffen eine Steuerung der Wundheilung, insbesondere dadurch, daß diese Wirkstoffe an die Gerüstsubstanz durch Transglutaminasen immobilisiert werden und entweder im immobilisierten Zustand ihre Wirkung ausüben können bzw. im Rahmen des Resorptions- und Remodellierungsprozesses freigesetzt werden.

Die im erfindungsgemäßen Arzneimittel enthaltenen allogenen Transglutaminasen, beispielsweise Faktor XIIIa, bewirken, daß die allogenen Proteaseinhibitoren kovalent an das Fibrin gebunden werden, wodurch es praktisch zu keiner Diffusion der Inhibitoren aus dem Wundgebiet kommt.

Eine bevorzugte Ausführungsform des erfindungsgemäßen Arzneimittels ist dadurch gekennzeichnet, daß alle Wirkstoffe in einer einzigen pharmazeutischen Zubereitung als allogene, provisorische extrazelluläre Matrix vorliegen, die beispielsweise in Form eines Gels ausgebildet ist. Das Arzneimittel ist in dieser Form direkt für die lokale Aufbringung verwendungsfähig.

Die Wirkstoffe können zweckmäßig auch in zwei oder mehreren getrennten pharmazeutischen Zubereitungen zum Vermischen vor oder während der Anwendung vorliegen, wobei das erhaltene Gemisch in flüssiger Form oder erst nach seiner Verfestigung aufgebracht werden kann. Die Zubereitungen können in flüssiger Form oder gefroren oder gefriergetrocknet zum Auftauen bzw. zur Rekonstitution vor dem Vermischen vorliegen.

Die pharmazeutischen Zubereitungen können beispielsweise mindestens zehn Minuten vor der Aufbringung auf eine Wundfläche vermischt werden, wonach es zur Verfestigung des Gemisches durch Ausbildung einer langsam resorbierbaren und remodellierbaren, allogenen, provisorischen extrazellulären Matrix kommt, die auf die Wundfläche aufgebracht wird. Die pharmazeutischen Zubereitungen können jedoch auch vermischt und das flüssige Gemisch unmittelbar danach, beispielsweise auch in Form eines Sprays, lokal aufgetragen werden, sodaß sich eine langsam resorbierbare, remodellierbare, allogene, provisorische extrazelluläre Matrix erst lokal ausbildet.

Es ist bevorzugt, daß die Konzentration an Fibrinogen und Proteaseinhibitoren im Arzneimittel so gewählt wird, daß das flüssige Gemisch mindestens 30 g/l Fibrinogen und 500 arbiträre Plasmaeinheiten Proteaseinhibitoren/l enthält.

Vorzugsweise liegen Fibrinogen und Thrombin jeweils in getrennten pharmazeutischen Zubereitungen vor, wobei die anderen Wirkstoffe unabhängig voneinander in einer oder beiden Zubereitungen und/oder in einer weiteren Zubereitung enthalten sind.

Durch entsprechende Zusammensetzung der thrombinhältigen pharmazeutischen Zubereitung wird es erfindungsgemäß möglich, daß noch bedeutende Mengen Thrombin bis einige Stunden nach Vermischen mit der fibrinogenhältigen Lösung und nach Verfestigung des Gemisches in dem gebildeten Fibrin generiert werden. Dies ist sowohl für die Stabilität als auch die Qualität der so ausgebildeten allogenen, provisorischen extrazellulären Matrix von großer Wichtigkeit.

Die in den erfindungsgemäßen Arzneimitteln vorliegenden pharmazeutischen Zubereitungen können vorteilhafterweise auch auf allogenen oder biokompatiblen Trägermaterialien aufgebracht sein, die einem Verfahren zur Virusabreicherung und/oder Virusinaktivierung unterzogen sind. Die Trägermaterialen können je nach Applikationszweck verschiedene Formen aufweisen. Die Herstellung eines anwendungsfertigen Arzneimittels kann beispielsweise durch Auftragen des noch flüssigen Gemisches der pharmazeutischen Zubereitungen auf das Trägermaterial erfolgen.

Eine bevorzugte Ausführungsform des erfindungsgemäßen Arzneimittels ist dadurch gekennzeichnet, daß es als weiteren Wirkstoff allogene Kollagene, die einem Verfahren zur Virusabreicherung und/oder Virusinaktivierung unterzogen sind, enthält. Die zusätzliche Verwendung von Kollagenen bei der Herstellung bzw. Ausbildung der allogenen, provisorischen extrazellulären Matrix führt zu einer beträchtlichen Erhöhung der biomechanischen Qualität.

Zweckmäßigerweise enthält das erfindungsgemäße Arzneimittel einen oder mehrere weitere, allogene, einem Verfahren zur Virusabreicherung und/oder Virusinaktivierung unterzogene Wirkstoffe, die aus der Gruppe Fibronektin, Vitronektin, Thrombospondin, Tenascin, Laminin und Proteoglykane ausgewählt sind. Durch den Zusatz von Substanzen wie z.B. Vitronektin kann noch eine Verbesserung der Wirkung bestimmter Proteaseinhibitoren erzielt werden.

Es ist weiters vorteilhaft, wenn das erfindungsgemäße Arzneimittel einen oder mehrere weitere, allogene, einem Verfahren zur Virusabreicherung und/oder Virusinaktivierung unterzogene Wirkstoffe enthält, die aus der Gruppe Wachstumsfaktoren, chemotaktische Substanzen, zellstimulierende und/oder proliferationsfördernde Enzyme und Enzyminhibitoren, proliferationshemmende Enzyme und Enzyminhibitoren, Zytokine und partikulär geformte Zellelemente ausgewählt sind. Die Anwendung von allogenen Enzyminhibitoren im Zusammenhang mit einem Wundverschluß bietet die Möglichkeit, insbesondere bei Hautulcera eine Dauerheilung zu erzielen.

Vorzugsweise enthält das erfindungsgemäße Arzneimittel zugesetzte allogene plasmatische bzw. aus Geweben gewonnene Enzyme, Zymogene und/oder Enzyminhibitoren, die einem Verfahren zur Virusabreicherung und/oder Virusinaktivierung unterzogen sind.

Je nach klinischer Erfordernis ist es zweckmäßig, daß dem erfindungsgemäßen Arzneimittel antiadhärente, antiphlogistische, antimikrobielle und/oder zytostatische Wirkstoffe zugesetzt sind, die erforderlichenfalls einem Verfahren zur Virusabreicherung und/oder Virusinaktivierung unterzogen sind. Der Zusatz antiphlogistischer und antiadhärenter, allogener Wirkstoffe, wie z.B. bestimmter Immunglobuline oder anderer mit diesen Wirkungen im Plasma vorkommender Wirksubstanzen, ermöglicht die Anwendung einer allogenen, provisorischen extrazellulären Matrix auch dort, wo postoperativ Adhäsionen befürchtet werden müssen.

Die sich bei Anwendung des Arzneimittels ausbildende oder als solches vorliegende allogene, provisorische extrazelluläre Matrix kann als Reservoir bzw. Depot für Substanzen fungieren, die langsam aus der Matrix freigesetzt werden sollen. Eine bevorzugte Ausführungsform des erfindungsgemäßen Arzneimittels ist daher dadurch gekennzeichnet, daß es zugesetzte Wirkstoffe enthält, die langsam resorbiert werden sollen und erforderlichenfalls einem Verfahren zur Virusabreicherung und/oder Virusinaktivierung unterzogen sind.

Vorteilhaft sind in der thrombinhältigen pharmazeutischen Zubereitung des erfindungsgemäßen Arzneimittels zusätzlich allogene Zymogene und/oder Enzyme der Gerinnungskaskade, die einem Verfahren zur Virusabreicherung und/oder Virusinaktivierung unterzogen sind, enthalten.

Vorzugsweise enthält das erfindungsgemäße Arzneimittel zusätzlich allogene partikuläre Zellelemente, Zellen und/oder Gewebe, die auf virussicheren Mikrosphären, welche aus allogenem Fibrin und/oder allogenem Kollagen und/oder allogenem Kollagen mit allogenem Fibrin bestehen, aufgebracht sein können.

Ein Nachteil der bisher bekannten fibrinogen- bzw. fibrinhältigen pharmazeutischen Präparationen ist die relativ große Fragilität des ausgebildeten Fibrins, selbst wenn sehr hochprozentige Fibrinogenlösungen verwendet werden.

Zwar wird durch die Anwesenheit von Faktor XIIIa - einer Transglutaminase, die sich in der pharmazeutischen Zubereitung aus dem darin enthaltenen Faktor XIII durch Thrombineinwirkung bildet, - das bei der Gerinnung entstehende Fibrin zu einem Gerüst quervernetzt, aber es müssen hohe Thrombin- und Faktor XIII-Konzentrationen noch im Fibrin vorhanden sein oder sich bilden, damit eine weitgehend komplette Reaktion zwischen den Vernetzungsstellen im Fibrin eintritt. Jedoch sind selbst solche Fibrine wegen der noch immer vorhandenen Fragilität schwierig zu verwenden, und ebenso zeigen die lokal ausgebildeten Fibrine nach wie vor geringe mechanische Festigkeit.

Eine bevorzugte Ausführungsform des erfindungsgemäßen Arzneimittels ist daher dadurch gekennzeichnet, daß die allogene, provisorische extrazelluläre Matrix durch Anwendung von Druck und/oder durch wasserentziehende Mittel verfestigt ist. Die Matrix kann dabei vor, während und/oder nach der Verfestigung durch Wasserentzug mit allogenen Transglutaminasen behandelt sein.

Solche allogenen Matrixen können durch ihre genügend mechanische Stabilität auch als artifizielle allogene Haut verwendet werden. Dadurch ist es auch möglich geworden, einen ausschließlich aus menschlichen Substanzen hergestellten Hautersatz herzustellen und zusätzlich in eine solche Präparation auch partikuläre Zellelemente, Zellen und Gewebe einzubringen, die neue medizinische Anwendungsmöglichkeiten ergeben.

Mit den erfindungsgemäßen pharmazeutischen Zubereitungen ist es auch möglich, an Körperstellen, wo ein starker Abbau von körpereigenem Gewebe stattgefunden hat, solche Stellen aufzufüllen und eine langfristige Heilung damit zu erreichen.

Konzentrierte Fibrinogenlösungen weisen eine Reihe von Nachteilen auf. Sie sind von einer geringen Lagerstabilität und müssen zur Lagerung tiefgefroren oder gefriergetrocknet werden und können durch Auftauen bzw. Wiederauflösen nicht immer befriedigend verwendbar gemacht bzw. rekonstituiert werden. Außerdem nimmt die Auflösung eines Fibirnogenlyophilisats geraume Zeit in Anspruch. Lösungsvermittler oder leicher lösliche Fibrinogene sind in den meisten Fällen zelltoxisch und daher für eine ungestörte Wundheilung nicht angebracht.

Die größte Schwierigkeit bei der Lagerung fibrinogenhältiger Produkte ist die Instabilität des Fibrinogens, da Spuren von Verunreinigungen mit Gerinnungsfaktoren eine langsame Umwandlung von Fibrinogen in unlösliches Fibrin bedingen, was dann die Anwendung eines solchen pharmazeutischen Präparates nicht mehr erlaubt.

Die vorliegende Erfindung betrifft daher auch ein Verfahren zur Herstellung einer fibrinogenhältigen Lösung, die als solche oder als Bestandteil von erfindungsgemäßen Arzneimitteln bei Kühlschranktemperatur oder Raumtemperatur lagerfähig ist, wobei die Fibrinogenlösung bzw. eine Fibrinogen-Fibronektinlösung aus gentechnologisch hergestelltem Fibrinogen oder aus Plasma gewonnenem Fibrinogen durch Fraktionierung mit Glyzin bei Temperaturen unter 0°C hergestellt wird.

Erfindungsgemäß hergestellte fibrinogenhältige Lösungen sind stabil und können im flüssigen Zustand mit oder ohne Stabilisatoren über zwei Jahre bei einer Temperatur von 4°-8°C oder eingefroren oder gefriergetrocknet gelagert werden, ohne daß dabei unlösliches Fibrin ausfällt oder sich Fibrinogenspaltprodukte durch die Entstehung von Plasmin während der Lagerung in einem Ausmaß bilden, daß die Gerinnbarkeit gestört wird. Solche Lösungen gerinnen weder nach Zusatz von Thromboplastin und Taipan Viper Venom, noch durch Zusatz von aktiviertem partiellem Thromboplastin. Bei Lagerung im tiefgefrorenen Zustand und nach dem Wiederauftauen der erfindungsgemäßen fibrinogenhältigen Lösungen werden keine Gerinnungsvorgänge induziert, und die erhaltenen Lösungen sind zumindest einige Stunden stabil. Gefriergetrocknete Präparationen sind leicht und vollständig mit entsprechenden Lösungsmitteln rekonstituierbar.

Es kann auch ein Arzneimittel hergestellt werden, das eine hochgereinigte fibrinogenhältige oder fibrinogen-fibronektinhältige Zubereitung enthält, deren aPTT und Taipan Viper Venom Prothrombinzeit bei 37°C nicht kürzer als 200 bzw. 300 Sekunden sind und die eine solche Stabilität aufweist, daß sie im flüssigen Zustand mit oder ohne Stabilisatoren über zwei Jahre bei einer Temperatur von 4°-8°C als Bestandteil von Arzneimitteln oder als solche gelagert oder eingefroren oder gefriergetrocknet gelagert werden kann, ohne daß dabei unlösliches Fibrin ausfällt oder sich Fibrinogenspaltprodukte durch die Entstehung von Plasmin während der Lagerung bilden.

Trotz Verwendung ausschließlich allogener Wirkstoffe kann es bei der Virusinaktivierung von aus allogenen Wirkstoffen zusammengesetzten Arzneimitteln zur Bildung von Neoantigenen kommen, entweder durch Interaktion zweier oder mehrerer Wirkstoffe des Arzneimittels während der Virusinaktivierung und/oder durch Interaktion mit Verunreinigungen, die in den Wirkstoffen noch enthalten sind.

Ein weiterer Nachteil bei der Virusinaktivierung von Wirkstoffgemischen ist die oft stark unterschiedliche Inaktivierung der einzelnen Wirkstoffe selbst, die eine Formulierung der Arzneimittel erschwert oder unmöglich macht.

Die Gewinnung von pathogenfreien, in den erfindungsgemäßen Arzneimitteln enthaltenen Wirkstoffen, kann daher eine Kombination von Abreicherungsverfahren und Inaktivierungsverfahren einschließlich Schritte zur Virusabreicherung und Virusinaktivierung umfassen, wobei zur Virusabreicherung Ultrazentrifugationen und Ultrafiltrationen einschließlich Nanofiltrationen und/oder Adsorptionen von Pathogenen bei Temperaturen unter 0°C verwendet werden und mindestens zwei unterschiedliche Virusinaktivierungsverfahren durchgeführt werden, bei denen Hitzeimpulsverfahren unter 3 Sekunden und/oder Intensivlaserimpulsbestrahlung mit oder ohne photodynamischen Substanzen und/oder Detergenzien gemeinsam mit hydrophoben Netzmitteln verwendet werden.

Durch dieses Verfahren können Wirkstoffe mit genügendem Reinheitsgrad vor ihrer Formulierung virusinaktiviert werden, wobei durch entsprechende Auswahl der Inaktivierungsverfahren sichergestellt wird, daß alle Wirkstoffe ohne zu große Verluste gleichartig virusinaktiviert sind. Auf diese Weise kann weitestgehend eine Induktion oder Auslösung von Allergien, Anaphylaxien und Autoimmunprozessen durch die diese Wirkstoffe enthaltenden Arzneimittel vermieden werden.

Es ist insbesondere auch eine besonders schonende Virusinaktivierung der in den erfindungsgemäßen Arzneimitteln enthaltenen Proteaseinhibitoren möglich.

Zur kovalenten Bindung von in den erfindungsgemäßen Arzneimitteln enthaltenen Wirkstoffen an eine biologische Matrix, werden zur enzymatischen Katalyse der Bindungsreaktion hohe Konzentrationen von allogenen, virusabgereicherten und/oder virusinaktivierten Transglutaminasen eingesetzt die über dem 10-fachen Konzentrationsbereich der aktivierten Konzentration des im Plasma vorkommenden Faktor XIIIa-Zymogens liegen.

Durch eine genügend hohe und lang anhaltende Thrombinkonzentration in der allogenen, provisorischen extrazellulären Matrix kann bei Anwesenheit von genügenden Mengen Faktor XIII genügend Faktor XIIIa gebildet werden, der einerseits die Quervernetzung der Grundsubstanz(en) Fibrin mit oder ohne Kollagen bewirkt, andererseits TAFI bei

Anwesenheit des TAFI-Zymogens generiert. TAFI spaltet vom Fibrin den Plasminrezeptor ab, wodurch eine große Stabilität der fibrinhältigen allogenen, provisorischen extrazellulären Matrix gegenüber fibrinolytischen Enzymen erzielt wird. Weiters ist es erfindungsgemäß durch eine hohe und lang anhaltende Transglutaminasekonzentration in der allogenen, provisorischen extrazellulären Matrix möglich, entsprechend virusinaktivierte allogene Protease-Inhibitoren, wie alpha₂-Antiplasmin, PAI-1 und andere, kovalent zu binden und somit ihre Diffusion aus der allogenen, provisorischen extrazellulären Matrix hintanzuhalten. Das Gleiche gilt auch für andere Wirkstoffe mit Proteincharakter, die sich mit Hilfe von Transglutaminasen kovalent an die Gerüstsubstanz binden lassen.

Zur Herstellung eines wasserarmen fibrinhältigen Gels mit einem Wassergehalt zwischen 20 bis 90% kann dem fibrinhältigen Gel durch Aufbringen von atoxischen, pharmazeutisch verwendbaren wasserentziehenden Mitteln, insbesondere Polyäthylenglykol, oder durch Einbringen des Gels in diese Mittel Wasser entzogen werden wird, wobei das Gel vor oder nach dem Wasserentzug mit Transglutaminasen behandelt werden kann.

Die Herstellung eines wasserarmen fibrinhältigen Gels mit einem Wassergehalt zwischen 20 bis 90% kann auch durch ein Verfahren erfolgen, bei welchem dem fibrinhältigen Gel durch Anwendung hoher Drucke, wobei diese zur Vermeidung einer Zerstörung des Gels stufenweise erhöht werden, Wasser entzogen wird. Das Gel kann dabei vor, während und/oder nach der Druckanwendung mit Transglutaminasen behandelt werden.

Zur Verfestigung eines fibrinhältigen Gels mit und ohne Entzug von Wasser wird das fibrinhältige Gel in eine oder mehrere metallionenhältige Lösungen, insbesondere Lösungen, die Zink- und Aluminiumionen in einer Konzentration von 0,01 bis 2 molar enthalten, eingelegt.

Es kann ferner ein gefriergetrocknetes, fibrinhältiges Gel zur Verfügung gestellt werden, das durch Zusätze von Weichmachern, insbesondere Glykol, vor seiner Verfestigung und Gefriertrocknung hergestellt ist.

Die vorstehend angeführten Verfahren zur Herstellung wasserarmer fibrinhältiger Gele sowie zur Verfestigung fibrinhältiger Gele können mit besonderem Vorteil zur Herstellung erfindungsgemäßer Arzneimittel eingesetzt werden.

Ein Nachteil der bisher verwendeten fibrinogenhältigen Zubereitungen bei der lokalen Auftragung fibrinogenhältiger Wirkstoffgemische ist ihre zu geringe Viskosität vor der Verfestigung. Dadurch kann das aufgetragene Gemisch leichter und schneller von der Auftragungsstelle abfließen, was einerseits zu einem Mehrverbrauch des Arzneimittels und andererseits zu einer unbefriedigenden Anwendung insbesondere im Zusammenhang mit chirurgischen Eingriffen führt, wenn eine Auftragung im Operationsfeld erforderlich ist.

Zur Herstellung einer hochviskosen, fibrinogenhältigen Lösung, kann eine sterile, virusabgereicherte und/oder virusinaktivierte Fibrinogenlösung pro Gramm Fibrinogen mit einer hundertstel bis zehntel Einheit sterilem, virusabgereichertem und/oder virusinaktiviertern Thrombin, das in einem möglichst kleinen Volumen gelöst ist, langsam und unter starkem Rühren unter sterilen Bedingungen versetzt Die durch das erfindungsgemäße Verfahren hergestellten hochviskosen, fibrinogenhältigen Lösungen können mit besonderem Vorteil zur Herstellung von erfindungsgemäßen Arzneimitteln verwendet werden.

Zur Bestimmung der Haftfähigkeit eines Fibrinclots im Wundbett können Fibrinclots mit steigenden Mengen von Proteaseinhibitoren an der Oberfläche von geeigneten Gewebekulturen, insbesondere menschlichen Fibroblasten, mit einem geringen Zusatz von einem oder mehreren Farbstoffen und/oder einer oder mehreren wasserunlöslichen Substanzen, die einen gefärbten bzw. opaleszenten Fibrinclot ergeben, ausgebildet werden und die Gewebekulturen durch leichtes Schütteln oder Rotieren in Bewegung gehalten werden, wobei die Zeit bestimmt wird, nach der sich die Fibringerinnsel von der Oberfläche der Gewebekultur lösen.

Mit diesem Verfahren ist es möglich, jene Mengen Proteaseinhibitoren in einer allogenen, provisorischen extrazellulären Matrix, wie sie beispielsweise in den erfindungsgemäßen Arzneimitteln vorliegt oder durch diese ausgebildet wird, zu ermitteln, die erforderlich sind, um eine solche Matrix über zumindest mehrere Tage am Ort ihrer Aufbringung zu belassen und somit eine Ablösung vom Wundbett wie auch eine dadurch verursachte Nachblutung zu verhindern.

### Beispiele

### 1. Beispiel

### Gewinnung einer stabilen Fibrinogenlösung.

1001 Plasma geeignet zur Gewinnung von Arzneimitteln oder die daraus mit Hilfe einer Kälte Äthanol-Fällung gewonnene Cohn Fraktion-I oder durch Einfrieren des Plasmas und vorsichtiges Auftauen gewonnenes Kältepräzipitat werden als Ausgangsmaterial verwendet.

Das Plasma wird als solches, die Cohn-Fraktion-I und das Kältepräzipitat nach dem Auflösen in etwa 20 Liter 0.9%iger NaCl und 0.1%igem Natriumzitratpuffer pH7 mit festem Glyzin mit einem Reinheitsgrad für pharmazeutische Zubereitungen bis zur Sättigung versetzt und dabei auf-2° C bis - 3 °C abgekühlt, mindestens 10 bis maximal 15 Stunden bei dieser Temperatur gelagert, von ungelöstem Glyzin abgetrennt und der fibrinogen-fibronektinhältige Niederschlag durch Zentrifugieren in einer Schnelllaufzentrifuge abgetrennt.

Die Überstände können für weitere Verarbeitung verwendet werden, um daraus noch weitere Plasmaproteine zu isolieren.

Der gallertartige Niederschlag wird aus dem Zentrifugenrotor entfernt, gelöst und wie vorher mit Glyzin gefällt. Das nach dem Zentrifugieren erhaltene Sediment wird in 0.1 % Zitrat gelöst und dieser Vorgang der Umfällung so oft wiederholt, bis eine Probe des erhaltenen Sediments in destilliertem Wasser aufgenommen, mit einem Fibrinogengehalt von 0.1% - 0.2% bei 37°C mit einem PTT Reagenz eine aPTT von nicht weniger als 200 sec. ergibt. Ebenso muß nach Zusatz von Thromboplastin und Taipan Viper Venom eine Gerinnungszeit erreicht werden, die nicht unter 300 sec. liegt.

Das letzte, zur weiteren Verarbeitung geeignete, wiederaufgelöste Präzipitat wird durch Diafiltration gegen eine 0.1%ige Zitratlösung von Glyzin weitgehend befreit und dabei auf 2% - 3% Proteingehalt konzentriert.

Das so gewonnene Fibrinogen enthält auch noch wesentliche Mengen Fibronektin. Falls erwünscht, kann Fibronektin durch Ausfällung bei -2° C bis - 3 °C mit 17%igem Glyzin abgetrennt werden. Dabei wird praktisch alles Fibrinogen gefällt und im Überstand befindet sich Fibronektin mit noch geringen Mengen Fibrinogen. Durch Sättigung mit Glyzin können beide Proteine gemeinsam ausgefällt und gewonnen werden. Allenfalls vorhandene, geringe Aktivitäten an Plasminogenaktivator oder Plasmin können durch Zusatz von geringen, niedermolekularen Inhibitoren, wie Epsilonaminokapronsäure oder Derivate in ihrer Wirkung aufgehoben werden.

Die Virusinaktivierung des hochgereinigten Fibrinogens oder des Fibrinogen-Fibronektin-Komplexes kann durch Versetzen des Plasmas der gelösten Cohn Fraktion-I oder des gelösten Kältepräzipitats mit Detergenzien und Netzmitteln erfolgen oder mit einem Hitzeimpuls- oder Laserlichtverfahren. Die virusinaktivierte Fibrinogenlösung kann als solche bei Kühlschranktemperatur bei 4° C bis 8° C gelagert, zur Lagerung tiefgefroren oder gefriergetrocknet werden.

### 2. Beispiel:

Herstellung einer Thrombinlösung mit thrombingenerierendem Potential.

Diese Thrombinlösung wird durch Vermischen zweier Lösungen zu gleichen Teilen, die aus zur Herstellung von Arzneimitteln für die Anwendung am Menschen geeignetem menschlichem Plasma gewonnen wurden, erhalten. Beide Lösungen sind pyrogenfrei, Caionenfrei und steril. Eine Lösung enthält virussicheres Thrombin in einer auszuwählenden Konzentration von 20 bis 2.500 Einheiten/ml, die andere enthält ein virussicheres Prothrombin-Gerinnungsfaktoren-Gemisch, das pro ml nach Ca-ionen-Zusatz mindestens 1.000 Thrombin-Einheiten generieren kann.

Diesem Gemisch wird ein hundertstel Volumsteil einer sterilen Lösung von 10% Polyäthylenglykol (pharmazeutische Reinheit) beigemischt und das Gemisch dann je nach gewünschter Dosierung in Durchstichfläschchen oder in Fertigspritzen verfüllt und tiefgefroren, allenfalls gefriergetrocknet, verschlossen, gelagert, etikettiert und verpackt.

### 3. Beispiel:

Herstellung von für den Menschen geeigneten Arzneimitteln zur lokalen Anwendung aus fibrinhältigen und thrombinhältigen pharmazeutischen Zubereitungen.

800 ml einer pyrogenfreien flüssigen Wirkstoffzubereitung nach Beispiel 1, die aus zur Herstellung von Arzneimitteln für den Menschen geeignetem menschlichem Plasma gewonnen wurde und einen Fibrinogengehalt von mindestens 6 % aufweist, werden zur Auflösung einer gefriergetrockneten, sterilen, pyrogenfreien, virusinaktivierten, aus zur Herstellung von Arzneimitteln für den Menschen geeignetem menschlichem Plasma gewonnenen pharmazeutischen Zubereitung verwendet, die 1.200 arbiträre, auf menschliches Plasma bezogene Einheiten PAI-1 oder eines anderen Serpins, bzw. Serpingemisches, das keine kollagenase- oder elastasehemmende Wirkung aufweist und ein Transglutaminasezymogengehalt äquivalent zu mindestens 4.000 Einheiten Faktor XIII enthält. Weiters enthält die Zubereitung 2 g CaCl₂.

Diese fibrinogenhältige pharmazeutische Zubereitung wird nun mit der im Beispiel 2 angegebenen thrombinhältigen Zubereitung nach deren Rekonstitution mit der entsprechenden Menge Wassers für Injektionszwecke vermischt, und zwar 4 Teile der fibrinogenhältigen Zubereitung mit 1 Teil der thrombinhältigen Zubereitung.

### 4. Beispiel:

Herstellung einer allogenen, provisorischen, extrazellulären Matrix als Arzneimittel.

Eine bestimmte Menge des Gemisches nach Beispiel 3 wird in eine gewünschte sterilisierte Form eingegossen und 5 Stunden unter sterilen Bedingungen zwischen 35° C und 37° C inkubiert. Die sich in der Form gebildete sterile, pyrogenfreie, virussichere, für die Verwendung beim Menschen allogene, extrazelluläre Matrix wird in sterile, wasserdampfdichte Polyäthylenhüllen verpackt, verschweißt, und bei Kühlschranktemperatur 4°C-8°C zur Freigabe nach den entsprechenden Qualitätskontrollen gelagert. Nach Etikettierung, Verpackung und Freigabe kann das Arzneimittel in den Verkehr gebracht werden.

### 5. Beispiel:

Herstellung einer allogenen, provisorischen, extrazellulären Matrix durch Vermischen dreier pharmazeutischer Zubereitungen, die als zusammengesetztes Arzneimittel angeboten werden.

Das Arzneimittel enthält drei pharmazeutischer Zubereitungen, die nach Vermischen eine allogene, provisorische, extrazelluläre Matrix ausbilden:
a. eine 6%ige Fibrinogenlösung nach Beispiel 3,
b. gefriergetrocknete Mischung von Transglutaminasezymogen, Serpin oder Serpingemisch, frei von kollagenase- und elastasehemmender Wirkung und Calciumchlorid in den in Beispiel 3 angegebenen Mengen.
c. eine gefriergetrocknete Thrombinlösung nach Beispiel 2.

Im Arzneimittel ist weiters Wasser für Injektionszwecke vorhanden, das benützt wird, um die thrombinhältige pharmazeutische Zubereitung gemäß Beispiel 2 aufzulösen, während die pharmazeutische Zubereitung, die das Transglutaminasezymogen und das Serpin enthält, in der 6%igen Fibrinogenlösung nach Beispiel 3 gelöst wird. Nach Vermischen der fibrinogenhältigen mit der thrombinhältigen Lösung wird vor der Gerinnung dieses Gemisches dieses entweder in eine gewünschte, sterile, pyrogenfreie Form eingebracht und nach Verfestigung aus der Form entfernt und lokal aufgebracht oder das noch flüssige Gemisch lokal auf die gewünschte Stelle bzw. die gewünschten Stellen aufgebracht, wobei sich erst dann lokal eine für den Menschen allogene, provisorische extrazelluläre Matrix ausbildet.

### 6. Beispiel:

Biomechanisch verstärkte, allogene, provisorische extrazelluläre Matrix unter Verwendung von allogenem Kollagen.

In das Gemisch der pharmazeutischen Zubereitungen nach Beispiel 4 und 5 kann noch fibriläres, steriles, pyrogenfreies, virussicheres, menschliches Kollagen eingebracht werden, und zwar zwischen 5 - 100 mg/ml, oder das Gemisch wird auf ein schnell saugfähiges, schaumförmiges Kollagenfließ aufgebracht, wobei der Thrombingehalt des Gemisches so eingestellt ist, daß die Fibrinbildung erst nach Ende des Aufsaugungsvorganges eintritt. Ein solches Fließ kann unmittelbar nach Einsetzen des Gerinnungsvorganges verwendet werden oder nach Inkubation von 5 Stunden zwischen 35° C und 37° C, steril verpackt, zum Fertigarzneimittel weiter- verarbeitet werden.

### 7. Beispiel:

Feststellung der erforderlichen Konzentrationen von nicht-Kollagenase oder -Elastase inhibierenden Serpinen.

Eine ausgewählte Fibroblastenzelllinie, deren Auswahl nach maximaler fibrinolytischer Aktivität erfolgt, wird mit einer Zelldichte von etwa 10⁷ Zellen pro ml in Gewebekulturflaschen mit einer Wachstumsfläche von mindestens 30 cm² in einer Menge eingebracht, daß pro cm² Gewebekulturfläche 10⁵ Zellen vorhanden sind. Nach Hinzufügung von mindestens 10 ml Gewebekulturmedium werden die Flaschen so lange inkubiert, bis sich ein kompletter Zellrasen ausgebildet hat. Falls erforderlich, wird in bestimmten Intervallen ein Wechsel des Mediums vorgenommen.
Nachdem ein vollständiger Zellrasen angewachsen ist, wird das Medium entfernt und zweimal mit mindestens je 20 ml Medium der Zellrasen gewaschen, das Medium weitestgehend aus der Flasche entfernt und mit Hilfe geeigneter Pipetten auf an der Außenseite der Flaschen vorgemerkten Stellen je 50 µl eines nachstehenden Gemisches auf den genau waagrecht gestellten Zellrasen aufgebracht und darnach die Gewebekulturflaschen verschlossen und 1 Stunde lang bei Raumtemperatur gelagert, darnach wieder wie vorher mit Medium aufgefüllt, verschlossen und auf eine Gewebekulturwippe bei einer Wippung von etwa 10 Wippen pro min. im Brutschrank bei 37° C gehalten, nach 30, 100, und 300 min. und darnach
alle 8 Stunden auf Ablösung der auf den Zellrasen aufgebrachten fibrinhältigen Clots inspiziert.

Die Aufbringung der nachstehenden Gemische erfolgt folgendermaßen:

Auf sechs vorbezeichneten Stellen wird Plasma, das vor der Vermischung mit Methylenblau in gewünschtem Ausmaß und tausend Einheiten Thrombin / ml Plasma versetzt worden war, aufgebracht. Versuchsweise werden Gemische nach Beispiel 3 hergestellt, die mit einer gewünschten Menge Phenolrot angefärbt, unterschiedliche Mengen plasmatischer oder gentechnologisch hergestellter Serpine enthalten. Von jeder Serpinkonzentration werden ebenfalls 6 x 50 µl des Gemisches auf vorbezeichnete Stellen aufgebracht. Nach Verfestigung der aufgebrachten Gemisch-proben wird wie oben angegeben verfahren.

Es werden nunmehr die Zeitpunkte bestimmt, zu denen sich die fibrinhältigen Proben von der Gewebekulturoberfläche ablösen. Die Ablösungszeiten der serpinhältigen Proben werden mit denen der Plasmaproben verglichen. Es werden vorteilhafter-weise jene Serpinkonzentrationen ausgewählt, die eine mindestens so lange Ablösezeit bewirken, wie sie bei den aus Plasma hergestellten und aufgebrachten Gerinseln beobachtet werden.

## Patentansprüche

1. Arzneimittel zur lokalen Anwendung zum Zwecke der Blutstillung und/oder des Wundverschlusses und/oder der Wundheilungsförderung, das als Wirkstoffe - konventionell aus allogenem Plasma oder Gewebe oder gentechnologisch hergestellt - Fibrinogen bzw. Fibrin, Thrombin und eine oder mehrere Transglutaminasen aufweist, **dadurch gekennzeichnet, daß** das Arzneimittel als weiteren Wirkstoff einen oder mehrerere Proteaseinhibitoren enthält, die aus der Gruppe der Serpine, die keine inhibierende Wirkung auf Kollagenasen und Elastasen besitzen, ausgewählt sind, wobei alle Wirkstoffe allogenen Ursprungs und einem Verfahren zur Virusabreicherung und/oder Virusinaktivierung unterzogen sind, mit der Maßgabe, daß die Virusinaktivierung des einen oder der mehreren Proteaseinhibitoren nicht in Gegenwart der anderen Wirkstoffe durchgeführt worden ist.

2. Arzneimittel nach Anspruch 1, **dadurch gekennzeichnet, daß** alle Wirkstoffe in einer einzigen pharmazeutischen Zubereitung als allogene provisorische extrazelluläre Matrix vorliegen.

3. Arzneimittel nach Anspruch 1, **dadurch gekennzeichnet, daß** die Wirkstoffe in zwei oder mehreren getrennten pharmazeutischen Zubereitungen zum Vermischen vor oder während der Anwendung vorliegen, wobei das erhaltene Gemisch in flüssiger Form oder erst nach seiner Verfestigung aufgebracht werden kann.

4. Arzneimittel nach Anspruch 3, **dadurch gekennzeichnet, daß** Fibrinogen und Thrombin jeweils in getrennten pharmazeutischen Zubereitungen vorliegen und die anderen Wirkstoffe unabhängig voneinander in einer oder beiden Zubereitungen und/oder in einer weiteren Zubereitung enthalten sind.

5. Arzneimittel nach einem der Ansprüche 2 bis 4, **dadurch gekennzeichnet, daß** die pharmazeutischen Zubereitungen auf allogenen oder biokompatiblen Trägermaterialien, die einem Verfahren zur Virusabreicherung und/oder Virusinaktivierung unterzogen sind, aufgebracht sind.

6. Arzneimittel nach einem der Ansprüche 1 bis 5, **dadurch gekennzeichnet, daß** es als weiteren Wirkstoff allogene Kollagene, die einem Verfahren zur Virusabreicherung und/oder Virusinaktivierung unterzogen sind, enthält.

7. Arzneimittel nach einem der Ansprüche 1 bis 6, **dadurch gekennzeichnet, daß** es einen oder mehrere weitere, allogene, einem Verfahren zur Virusabreicherung und/oder Virusinaktivierung unterzogene Wirkstoff(e) enthält, die aus der Gruppe Fibronektin, Vitronektin, Thrombospondin, Tenascin, Laminin und Proteoglykane ausgewählt sind.

8. Arzneimittel nach einem der Ansprüche 1 bis 7, **dadurch gekennzeichnet, daß** es einen oder mehrere weitere, allogene, einem Verfahren zur Virusabreicherung und/oder Virusinaktivierung unterzogene Wirkstoff(e) enthält, die aus der Gruppe Wachstumsfaktoren, chemotaktische Substanzen, zellstimulierende und/oder proliferationsfördernde Enzyme und Enzyminhibitoren, proliferationshemmende Enzyme und Enzyminhibitoren, Zytokine und partikulär geformte Zellelemente ausgewählt sind.

9. Arzneimittel nach einem der Ansprüche 1 bis 8, **dadurch gekennzeichnet, daß** es zugesetzte allogene plasmatische bzw. aus Geweben gewonnene Enzyme, Zymogene und/oder Enzyminhibitoren enthält, die einem Verfahren zur Virusabreicherung und/oder Virusinaktivierung unterzogen sind.

10. Arzneimittel nach einem der Ansprüche 1 bis 9 **dadurch gekennzeichnet, daß** es zugesetzte antiadhärente und/oder antiphlogistische Wirkstoffe enthält, die erforderlichenfalls einem Verfahren zur Virusabreicherung und/oder Virusinaktivierung unterzogen sind.

11. Arzneimittel nach einem der Ansprüche 1 bis 10, **dadurch gekennzeichnet, daß** es zugesetzte antimikrobielle und/oder zytostatische Wirkstoffe enthält, die erforderlichenfalls einem Verfahren zur Virusabreicherung und/oder Virusinaktivierung unterzogen sind.

12. Arzneimittel nach einem der Ansprüche 1 bis 11, **dadurch gekennzeichnet, daß** es zugesetzte Wirkstoffe enthält, die langsam resorbiert werden sollen und erforderlichenfalls einem Verfahren zur Virusabreicherung und/oder Virusinaktivierung unterzogen sind.

13. Arzneimittel nach einem der Ansprüche 1 bis 12, **dadurch gekennzeichnet, daß** in der thrombinhältigen pharmazeutischen Zubereitung zusätzlich allogene Zymogene und/oder Enzyme der Gerinnungskaskade, die einem Verfahren zur Virusabreicherung und/oder Virusinaktivierung unterzogen sind, enthalten sind.

14. Arzneimittel nach einem der Ansprüche 1, 2 und 5 bis 13, **dadurch gekennzeichnet, daß** es zusätzlich allogene partikuläre Zellelemente, Zellen und/oder Gewebe enthält.

15. Arzneimittel nach Anspruch 14, **dadurch gekennzeichnet, daß** die allogenen partikulären Zellelemente, Zellen und/oder Gewebe auf virussicheren Mikrosphären aufgebracht sind, die aus allogenem Fibrin und/oder allogenem Kollagen und/oder allogenem Kollagen mit allogenem Fibrin bestehen.

16. Arzneimittel nach einem der Ansprüche 2 und 5 bis 15, **dadurch gekennzeichnet, daß** die allogene provisorische extrazelluläre Matrix durch Anwendung von Druck und/oder durch wasserentziehende Mittel verfestigt ist.

17. Arzneimittel nach Anspruch 16, **dadurch gekennzeichnet, daß** die allogene provisorische extrazelluläre Matrix vor, während und/oder nach der Verfestigung durch Wasserentzug mit allogenen Transglutaminasen behandelt ist.

18. Verfahren zur Herstellung einer fibrinogenhältigen Lösung, die als Bestandteil eines Arzneimittels nach einem der Ansprüche 1 bis 17 bei Kühlschranktemperatur oder Raumtemperatur lagerfähig ist, **dadurch gekennzeichnet, daß** die Fibrinogenlösung bzw. eine Fibrinogen-Fibronektinlösung aus gentechnologisch hergestelltem Fibrinogen oder aus Plasma gewonnenem Fibrinogen durch Fraktionierung mit Glyzin bei Temperaturen unter 0°C hergestellt wird.

## Claims

1. A medicament for topical application intended for stopping bleedings and/or closing wounds and/or promoting wound healing, which as active substances - produced conventionally of allogenic plasma or tissue or by way of genetic engineering - contains fibrinogen or fibrin, respectively, thrombin and one or several transglutaminase(s), **characterized in that** the medicament, as a further active substance, contains one or several protease inhibitor(s) selected from the group consisting of serpins that do not have inhibiting effects on collagenases and elastases, all of the active substances being of allogenic origin and having been subjected to a process for virus depletion and/or virus inactivation, with the proviso that the virus inactivation of said one or several protease inhibitor(s) has not been carried out in the presence of the other active substances.

2. A medicament according to claim 1, **characterized in that** all of the active substances are present as an allogenic provisional extracellular matrix in a single pharmaceutical preparation.

3. A medicament according to claim 1, **characterized in that** the active substances are present in two or several separate pharmaceutical preparations to be mixed prior to or during application, wherein the mixture obtained may be applied in the liquid form or only after its solidification.

4. A medicament according to claim 3, **characterized in that** fibrinogen and thrombin are each present in separate pharmaceutical preparations and the remaining active substances, independent of one another, are contained in one or both preparations and/or in a further preparation.

5. A medicament according to any of claims 2 to 4, **characterized in that** the pharmaceutical preparations are applied on allogenic or biocompatible carrier materials subjected to a process for virus depletion and/or virus inactivation.

6. A medicament according to any of claims 1 to 5, **characterized in that** it contains, as further active substances, allogenic collagens subjected to a process for virus depletion and/or virus inactivation.

7. A medicament according to any of claims 1 to 6, **characterized in that** it contains one or several further allogenic active substance(s) subjected to a process for virus depletion and/or virus inactivation and selected from the group consisting of fibronectin, vitronectin, thrombospondin, tenascin, laminin and proteoglycans.

8. A medicament according to any of claims 1 to 7, **characterized in that** it contains one or several further allogenic active substance(s) subjected to a process for virus depletion and/or virus inactivation and selected from the group consisting of growth factors, chemotactic substances, cell stimulating and/or proliferation enhancing enzymes and enzyme inhibitors, proliferation inhibiting enzymes and enzyme inhibitors, zytokins and particulately formed cell elements.

9. A medicament according to any of claims 1 to 8, **characterized in that** it contains additions of allogenic plasmatic enzymes, or enzymes obtained from tissues, zymogens and/or enzyme inhibitors subjected to a process for virus depletion and/or virus inactivation.

10. A medicament according to any of claims 1 to 9, **characterized in that** it contains additions of antiadherent and/or antiphlogistic agents subjected to a process for virus depletion and/or virus inactivation, if necessary.

11. A medicament according to any of claims 1 to 10, **characterized in that** it contains additions of antimicrobial and/or cytostatic agents subjected to a process for virus depletion and/or virus inactivation, if necessary.

12. A medicament according to any of claims 1 to 11, **characterized in that** it contains additions of active substances that are to be resorbed slowly and, if necessary, have been subjected to a process for virus depletion and/or virus inactivation.

13. A medicament according to any of claims 1 to 12, **characterized in that** allogenic zymogens and/or enzymes of the coagulation cascade that have been subjected to a process for virus depletion and/or virus inactivation are additionally contained in the thrombin-containing pharmaceutical preparation.

14. A medicament according to any of claims 1, 2 and 5 to 13, **characterized in that** it additionally contains allogenic particulate cell elements, cells and/or tissues.

15. A medicament according to claim 14, **characterized in that** the allogenic particulate cell elements, cells and/or tissues are applied on virus-safe microspheres consisting of allogenic fibrin and/or allogenic collagen and/or allogenic collagen plus allogenic fibrin.

16. A medicament according to any of claims 2 and 5 to 15, **characterized in that** the allogenic provisional extracellular matrix is solidified by the application of pressure and/or by means of dehydrating agents.

17. A medicament according to claim 16, **characterized in that** the allogenic provisional extracellular matrix is treated with allogenic transglutaminases prior to, during and/or after solidification by dehydration.

18. A process for preparing a fibrinogen-containing solution which, as a component of a medicament according to any of claims 1 to 17, is storable at refrigerator temperature or room temperature, **characterized in that** the fibrinogen solution or a fibrinogen-fibronectin solution is prepared of fibrinogen produced by genetic engineering or of fibrinogen obtained from plasma by fractionation with glycine at temperatures of below 0°C.

## Revendications

1. Médicament pour application locale, dans un but hémostatique et/ou de fermeture d'une blessure et/ou de favoriser la guérison d'une blessure, présentant, en tant que principaux actifs - préparés de façon classique, à partir de plasma allogène ou de tissus ou par une technologie de génie génétique - du fibrinogène, respectivement de la fibrine, de la trombine et un ou plusieurs transglutaminases, **caractérisé en ce que** le médicament contient, à titre d'autre principe actif un ou plusieurs inhibiteurs de protéase, sélectionnés dans le groupe de la serpine, n'ayant aucun effet inhibant sur les collagénases et les élastases, sachant que tous les principes actifs sont d'origine allogène et sont soumis à un procédé d'administration virale et/ou d'inactivation virale, avec indication que l'inactivation virale d'un ou plusieurs inhibiteurs de protéase n'est pas effectuée en présence des autres principes actifs.

2. Médicament selon la revendication 1, **caractérisé en ce que** tous les principes actifs sont présents dans une préparation pharmaceutique unique, en tant que matrice extra cellulaire provisoire allogène.

3. Médicament selon la revendication 1, **caractérisé en ce que** les principes actifs sont présents en deux préparations pharmaceutiques séparées, ou plus, pour mélange avant ou pendant l'application, le mélange obtenu pouvant être appliqué sous forme liquide, ou bien ensuite, après sa solidification.

4. Médicament selon la revendication 3, **caractérisé en ce que** le fibrinogène et la trombine sont respectivement présents dans des préparations pharmaceutiques séparées et les autres principes actifs sont contenus indépendamment les uns des autres, en une ou les deux préparations et/ou en une autre préparation.

5. Médicament selon l'une des revendications 2 à 4, **caractérisé en ce que** les préparations pharmaceutiques sont appliquées sur des matériaux support allogènes ou biocompatibles, soumis à un procédé d'administration virale et/ou d'inactivation virale.

6. Médicament selon l'une ou plusieurs des revendications 1 à 5, **caractérisé en ce qu'**il contient, à titre d'autre principe actif, des collagènes allogènes soumis à un procédé d'administration virale et/ou d'inactivation virale.

7. Médicament selon l'une des revendications 1 à 6, **caractérisé en ce qu'**il contient un ou plusieurs autres principes actifs, allogènes, soumis à un procédé d'administration virale et/ou d'inactivation virale, sélectionnés dans le groupe de la fibronectine, vitronectine, thrombospondine, tenascine, laminine et prothéoglycane.

8. Médicament selon l'une des revendications 1 à 7, **caractérisé en ce qu'**il contient un ou plusieurs autres principaux actifs allogènes, soumis à un procédé d'administration virale et/ou d'inactivation virale, sélectionné dans le groupe des facteurs de croissance, substances chémotactiques, des enzymes stimulateurs cellulaires et/ou favorisant la profilération, et des inhibiteurs d'enzyme, des enzymes entravant la profilération et des inhibiteurs d'enzyme, de la zytocine et des éléments cellulaires de forme particulaire.

9. Médicament selon l'une des revendications 1 à 8, **caractérisé en ce qu'**il contient des enzymes, des zymogènes, et/ou des inhibiteurs d'enzyme, ajoutés, allogènes, plasmatiques, respectivement obtenus à partir de tissus.

10. Médicament selon l'une des revendications 1 à 9, **caractérisé en ce qu'**il contient principes actifs anti-adhérant et/ou anti-phlogistiques qui, le cas échéant, sont soumis à un procédé d'administration virale et/ou d'inactivation virale.

11. Médicament selon l'une des revendications 1 à 10, **caractérisé en ce qu'**il contient des principes actifs ajoutés, anti-microbiens et/ou cytostatiques, en cas de nécessité soumis à un procédé d'administration virale et/ou d'inactivation virale.

12. Médicament selon l'une des revendications 1 à 11, **caractérisé en ce qu'**il contient des principes actifs ajoutés, devant être résorbés lentement et, en cas de nécessité, soumis à un procédé d'administration virale et/ou d'inactivation virale.

13. Médicament selon l'une des revendications 1 à 12, **caractérisé en ce que**, dans la préparation pharmaceutique, contenant de la trombine, sont contenus en plus des zymogènes allogènes et/ou des enzymes de la cascade de coagulation, soumis à un procédé d'administration virale et/ou d'inactivation virale.

14. Médicament selon l'une des revendications 1, 2 et 5 à 13, **caractérisé en ce qu'**il contient, à titre supplémentaire, des éléments cellulaires particulaires, des cellules, des cellules et/ou des tissus allogènes.

15. Médicament selon la revendication 14, **caractérisé en ce que** les éléments cellulaires particulaires, les cellules et/ou les tissus allogènes, sont appliqués sur des microsphères viralement sûres, formées de fibrine allogène et/ou de collagène allogène et/ou de collagène allogène, avec de la fibrine allogène.

16. Médicament selon l'une des revendications 2 et 5 à 15, **caractérisé en ce que** la matrice extracellulaire provisoire allogène est renforcée par application de pression et/ou par des moyens extracteurs de l'eau.

17. Médicament selon la revendication 7, **caractérisé en ce que** la matrice extracellulaire provisoire allogène est traitée avant, pendant et/ou après la consolidation par extraction de l'eau, avec des transglutaminases allogènes.

18. Procédé de préparation d'une solution contenant des fibrinogènes, stockable à la température d'un réfrigérateur ou à la température ambiante en tant que composant d'un médicament selon l'une des revendications 1 à 17, **caractérisé en ce que** la solution de fibrinogène, respectivement une solution fibrinogène - fibronectine, est préparée à partir de fibrinogène produite par génie génétique, ou à partir de fibrinogène obtenu à partir de plasma, par fractionnement avec de la glycine, à des températures inférieures à 0°C.
